# EUROPÄISCHE PATENTSCHRIFT

(11) **EP 3 208 609 B1**
(45) Veröffentlichungstag und Bekanntmachung des Hinweises auf die Patenterteilung: **11.03.2020**
(21) Anmeldenummer: 16200346.1
(22) Anmeldetag: 23.11.2016
(51) Int. Cl.: G01N 25/48, G01N 33/00, H01L 35/32, G01N 15/08, A61B 5/00, G01N 33/36

(54) **VERFAHREN UND VORRICHTUNG ZUM MESSEN EINER ÜBERGANGSRATE EINES PHASENÜBERGANGS**
METHOD AND DEVICE FOR MEASURING A TRANSITION RATE OF A PHASE TRANSITION
PROCÉDÉ ET DISPOSITIF DE MESURE D'UN TAUX DE CHANGEMENT DE PHASE

(30) Priorität: 02.12.2015 DE 102015120899
(43) Veröffentlichungstag der Anmeldung: 23.08.2017
(73) Patentinhaber: Liu, Jin-Chen, 85221 Dachau (DE)
(72) Erfinder: Liu, Jin-Chen, 85221 Dachau (DE)
(74) Vertreter: Grünecker Patent- und Rechtsanwälte PartG mbB

(56) Entgegenhaltungen:
- DE-A1-102005 061 411
- US-A1- 2006 070 650
- SUSAN STEINBERG ET AL: "A Gauge to Measure Mass Flow Rate of Sap in Stems and Trunks of Woody Plants", JOURNAL OF THE AMERICAN SOCIETY FOR HORTICULTURAL SCIENCE, Bd. 114, Nr. 3, Mai 1989 (1989-05), Seiten 466-472, XP055361027,
- HAINES F M: "Transpiration and Pressure Deficit. III. Observations by the Thermopile Method", ANNALS OF BOT, ACADEMIC PRESS, LONDON, GB, Bd. OS-50, Nr. 1, Januar 1936 (1936-01), Seiten 1-22, XP008183973, ISSN: 0305-7364, DOI: 10.1093/OXFORDJOURNALS.AOB.A090575

## Beschreibung

Die vorliegende Erfindung betrifft ein Verfahren und eine Vorrichtung zum Messen einer Übergangsrate zwischen einer festen oder flüssigen Phase eines Materials und einer gasförmigen Phase des Materials. Besonders vorteilhaft lässt sich die Erfindung auf das Messen einer Übergangsrate eines Phasenübergangs, insbesondere eines flüssig-gasförmig Phasenübergangs, von Wasser anwenden. Die Erfindung kann aber auch zum Messen der Übergangsrate zwischen Phasen anderer Materialien, wie z.B. Alkohol, angewandt werden.

Aus der Geschwindigkeit einer Phasenänderung eines Materials lassen sich eine Reihe von physikalischen Größen bestimmen. Die Erfindung betrifft daher auch Anwendungen des Verfahrens und der Vorrichtung zum Messen der Übergangsrate des Phasenübergangs.

Aus der US 7,631,538 B2 ist eine Vorrichtung zum Messen des Wasserdampfflusses von einer Oberfläche bekannt. Im Speziellen handelt es sich bei der Oberfläche um menschliche Haut, deren Wasserverlustrate bestimmt werden soll. Es wird ein erstes Ende eines Röhrchens an der Oberfläche aufgesetzt. Mittels zweier in Erstreckungsrichtung des Röhrchens voneinander beabstandeter Luftfeuchtigkeitssensoren wird der Feuchtigkeitsgradient in dem Röhrchens gemessen. Mit den erhaltenen Daten kann dann der Wasserdampffluss von der Oberfläche weg berechnet werden.

Aus der US 4,312,218 A ist ein sogenanntes Porometer zur Messung der Transpiration von Pflanzenblättern bekannt. Ein zu vermessendes Blatt wird in eine Kammer eingeschlossen. Dann wird ein Strom trockener Luft in die Kammer so gesteuert, dass die Luftfeuchtigkeit in der Kammer konstant bleibt. Aus der Flussgeschwindigkeit der Luft kann schließlich die Transpiration des Blattes bestimmt werden.

Die beschriebenen bekannten Verfahren basieren jeweils auf einer dynamischen Messung der Wasserkonzentration in der Gasphase, um einen flüssig-gasförmig Phasenübergang von Wasser zu charakterisieren. Eine solche indirekte Bestimmung der Übergangsrate kann sehr empfindlich auf äußere Gegebenheiten reagieren und relativ aufwendig sein.

Aus dem Artikel S. Steinberg et al "A Gauge to Measure Mass Flow Rate of Sap in Stems and Trunks of Woody Plants", Journal of the American Society for Horticultural Science, Bd. 114, Nr. 3, Mai 1989 ist es bekannt, Thermoketten einzusetzen, um lokale Temperaturunterschiede an einem Baumstamm zu bestimmen, um so den Fluss von Saft in dem Stamm verfolgen zu können.

Aus der DE 10 2005 061 411 A1 ist ein Verfahren zum Herstellen eines auf Thermoketten basierenden Sensorelements zum Einsatz in Infrarotsensoren bekannt.

Die US 2006/0 070 650 A1 beschreibt den Einsatz von Thermoketten zum Überwachen einer lokalen Temperatur an Wunden.

Der Artikel F. M. Haines "Transpiration and Pressure Deficit. III. Observations by the Thermopile Method", Annals of Botany, Bd. 0S-50, Nr. 1, Januar 1936 beschreibt den Einsatz von Thermoketten zum Vermessen einer Transpiration einer Pflanze.

Es ist Aufgabe der vorliegenden Erfindung, eine möglichst einfache, zuverlässige und direkte Messung einer Übergangsrate eines Phasenübergangs zwischen einer festen oder flüssigen und einer gasförmigen Phase eines Materials zu ermöglichen.

Diese Aufgabe wird durch den Gegenstand der Ansprüche 1, 6 und 10 gelöst. Die abhängigen Ansprüche geben vorteilhafte Ausführungsformen der Erfindung an.

Die Erfindung stellt ein Verfahren und eine Vorrichtung zum Messen einer Übergangsrate zwischen einer ersten Phase eines Materials und einer zweiten Phase des Materials bereit. In der ersten Phase liegt das Material fest oder flüssig vor. In der zweiten Phase liegt das Material gasförmig vor.

Die Erfindung erlaubt ein Messen der Übergangsrate direkt an einer Grenzfläche zwischen der ersten und der zweiten Phase. Mit Hilfe des erfindungsgemäßen Verfahrens und/oder der Vorrichtung kann eine tatsächliche Übergangsrate zwischen den beiden Phasen des Materials als physikalische Größe, beispielsweise in der Einheit [mol/(m^2^{∗}s)] (bezogen auf den vermessenen Bereich der Grenzfläche), bestimmt werden. Dies kann je nach Anwendung aber relativ aufwändig sein, da hierzu Kalibrierungsmessungen oder Referenzwerte unter bekannten Bedingungen notwendig sein können. Für einige Anwendungen ist es ausreichend, wenn eine Größe (beispielsweise die nachfolgend beschriebene Thermospannung) gemessen wird, die beispielsweise proportional zu der tatsächlichen Übergangsrate ist, aus der sich die Übergangsrate bei Durchführen entsprechender Kalibrierungsmessungen bestimmen lässt, oder die ein Vergleichen verschiedener gemessener Übergangsraten erlaubt. Im Sinne der vorliegenden Erfindung kann auch das Bestimmen einer solchen Größe als "Messen der Übergangsrate" zwischen den Phasen gelten, ohne dass die eigentliche Übergangsrate explizit quantifiziert wird.

Die vorliegende Erfindung basiert auf einem Messen einer an der Grenzfläche zwischen den Phasen aufgrund einer latenten Wärme (Verdunstungs- oder Kondensationswärme bzw. Sublimations- oder Resublimationswärme) des Phasenübergangs vorliegenden Temperaturdifferenz.

Zum Messen dieser Temperaturdifferenz wird gemäß dem erfindungsgemäßen Verfahren eine Thermokette an der Grenzfläche zwischen der ersten und der zweiten Phase bereitgestellt.

Thermoketten nutzen den thermoelektrischen Effekt zur Temperaturmessung und sind beispielsweise von Anwendungen in Strahlungsdetektoren bekannt. Sie umfassen mehrere Thermoelemente, die thermisch parallel und elektrisch in Reihe geschaltet sind. Die Summe der von den einzelnen Thermoelementen generierten Teilspannungen ist zwischen den beiden Enden der Thermokette als Thermospannung mittels einer Spannungsmessvorrichtung abgreifbar. Die Thermokette umfasst eine Vielzahl von Leiterelementen, beispielsweise in Form einzelner Drähte oder Drahtsegmente. Es sind Leiterelemente aus zwei verschiedenen Materialien, beispielsweise Konstantan und Kupfer oder Tellur und Antimon, vorgesehen, die abwechselnd hintereinander zum Bilden der Thermokette angeordnet sind. Aufeinanderfolgende Leiterelemente verschiedenen Materials sind jeweils an Leiterübergängen miteinander verbunden und bilden ein Thermoelement der Thermokette. Die Thermokette umfasst einen ersten Bereich, der jeden zweiten der Leiterübergänge umfasst, und einen zweiten Bereich, der die übrigen Leiterübergänge umfasst. Vorzugsweise liegen sich der erste Bereich und der zweite Bereich der Thermokette gegenüber, insbesondere verlaufen sie parallel zueinander. Die von der Thermokette ausgegebene Thermospannung repräsentiert eine Temperaturdifferenz zwischen dem ersten und dem zweiten Bereich der Thermokette.

Erfindungsgemäß wird die Thermospannung der an der Grenzfläche zwischen den Phasen bereitgestellten Thermokette gemessen. Diese Thermospannung repräsentiert eine aufgrund der latenten Wärme des Phasenübergangs vorliegende Temperaturdifferenz im Bereich der Phasengrenze und lässt daher auf die Übergangsrate des Phasenübergangs schließen. Vorteilhafterweise umfasst die Thermokette eine große Anzahl von Thermoelementen (also viele Leiterübergänge). Da sich die von den einzelnen, in Reihe geschalteten Thermoelementen generierten Teilspannungen aufaddieren, kann so die durch die latente Wärme des Phasenübergangs erzeugte, typischerweise sehr geringe Temperaturdifferenz detektiert werden. Als vorteilhaft hat es sich erwiesen, wenn die Thermokette zumindest 10, 50, 100, 500, 1000 oder mehr als 1000 Leiterübergänge umfasst. Es erschließt sich, dass die in der vorliegenden Erfindung verwendete Thermokette aus einer Mehrzahl von über Verbindungselemente hintereinandergeschalteten kürzeren Thermoketten zusammengesetzt sein kann.

Der Zusammenhang zwischen der gemessenen Thermospannung und der Übergangsrate des Phasenübergangs ist z.B. von dem zu vermessenden Material und der Orientierung der Thermokette in Bezug auf die Grenzfläche abhängig. Über Kalibrierungsmessungen an bekannten Systemen können diese und weitere Abhängigkeiten berücksichtigt werden, um aus der Thermospannung auf die Übergangsrate zwischen den Phasen zu schließen. Aber auch ohne explizite Bestimmung der Übergangsrate können bereits aus der gemessenen Thermospannung Aussagen getroffen werden, beispielsweise, wenn Übergangsraten verschiedener Systeme verglichen werden sollen. Eine hohe gemessene Thermospannung (hohe Temperaturdifferenz zwischen dem ersten und dem zweiten Bereich der Thermokette) lässt auf eine hohe Übergangsrate schließen. Umgekehrt kann eine geringe gemessene Thermospannung (geringe Temperaturdifferenz zwischen dem ersten und dem zweiten Bereich der Thermokette) auf eine geringe Übergangsrate schließen lassen.

Gemäß einer Ausführungsform wird die gemessene Thermospannung in einem Verfahren zum Bestimmen eines Druckpotentials p einer der beiden Phasen verwendet. Das Druckpotential p ist ein Maß dafür, wie viel Energie aufgewendet werden muss oder erhalten wird, wenn der Phase eine bestimmte Menge des Materials zugeführt wird. Dabei gibt ein negatives Druckpotential p beim Zuführen von weiterem Material freiwerdende Energie an und ein positives Druckpotential p gibt beim Zuführen von weiterem Material aufzuwendende Energie an. Ohne äußere Einflüsse diffundiert das Material also typischerweise von einem Bereich hohen Druckpotentials in einen Bereich niedrigen Druckpotentials. Das Druckpotential einer Phase zeigt gewissermaßen die Aufnahmebereitschaft für weiteres Material an. Besonders viele Anwendungen hat die Messung des Druckpotentials p von Wasser, welches oftmals "Wasserpotential" genannt wird. Beispielsweise besteht Bedarf an der Bestimmung des Wasserpotentials verschiedener Materiezusammensetzungen, wie z.B. Böden, Baumaterial, Pflanzen, Lebensmittel, Medikamente oder Textilien.

Das Verfahren zur Bestimmung des Druckpotentials p einer der beiden Phasen unter Verwendung der gemessenen Thermospannung basiert auf dem sogenannten Darcy-Gesetz. Dieses besagt, dass die Übergangsrate eines Materials zwischen einem ersten Gebiet und einem zweiten Gebiet direkt proportional zu der Differenz der Druckpotentiale des Materials in den beiden Gebieten ist. Wenn in dem ersten Gebiet das Material in der ersten Phase vorliegt und in dem zweiten Gebiet in der zweiten Phase, lässt sich das Darcy-Gesetz auf die Übergangsrate zwischen der ersten und der zweiten Phase anwenden: Vt = k^{∗}(p2 - p1). Dabei ist Vt die Übergangsrate von der ersten Phase in die zweite Phase (kann negativ sein), p1 das Druckpotential der ersten Phase, p2 das Druckpotential der zweiten Phase und k ein Proportionalitätsfaktor. Die Übergangsrate Vt kann aus der gemessenen Thermospannung bestimmt werden. Der Proportionalitätsfaktor k ist von der Permeabilität der Grenzfläche abhängig und kann durch geeignete Referenzmessungen bestimmt werden. Damit kann mithilfe des Darcy-Gesetzes das Druckpotential p1, p2 der ersten Phase oder der zweiten Phase basierend auf einem bekannten oder gemessenen Wert des Druckpotentials p1, p2 der anderen Phase des Materials bestimmt werden, wenn die Thermospannung gemessen wurde. Soll das Druckpotential p1 der ersten Phase bestimmt werden, kann der dafür benötigtet Wert des Druckpotentials p2 der gasförmigen Phase des Materials beispielsweise einfach bestimmt werden. Dazu sind nur eine Temperatur- und eine Feuchtemessung notwendig. Das Druckpotential in der Gasphase kann dann als p2 = RT/Ṽ^{∗}ln(ϕ) bestimmt werden, wobei R die Universelle Gaskonstante, T die Temperatur in K, Ṽ das Molvolumen des Materials im flüssigen Zustand und ϕ die relative Feuchtigkeit in der Gasphase ist. Die relative Feuchtigkeit in der Gasphase ϕ ist dabei für alle Materialien analog zu der "relativen Luftfeuchtigkeit" im Fall von Wasser definiert.

Gemäß einer anderen Ausführungsform kann die gemessene Thermospannung zum Bestimmen einer Permeabilität einer Trennschicht, insbesondere einer Folie oder einer Textilschicht, verwendet werden. Dabei wird die Trennschicht derart positioniert, dass sie während der Messung der Thermospannung zwischen der ersten Phase und der zweiten Phase vorliegt, so dass das Material beim Phasenübergang die Trennschicht passieren muss. Die Permeabilität der Trennschicht kann aus dem Proportionalitätsfaktor k aus dem Darcy-Gesetz bestimmt werden. Die Permeabilität der Trennschicht kann also anhand der gemessenen Thermospannung bestimmt werden, wenn zudem die Werte der Druckpotentiale p1, p2 der beiden Phasen des Materials bekannt sind. Besonders einfach lässt sich eine Permeabilitätsmessung vornehmen, wenn als erste Phase eine Flüssigkeit mit bekanntem Druckpotential p1 verwendet wird, und das Druckpotential p2 in der Gasphase, wie oben beschrieben, über eine Temperatur- und eine Feuchtemessung bestimmt wird.

Je nach Anwendung sind verschiedene Varianten der Positionierung der Thermokette an der Grenzfläche zwischen den Phasen denkbar. Es kann in jedem Fall vorteilhaft sein, wenn die Thermokette möglichst nahe an der Grenzfläche positioniert wird, da die latente Wärme an der Grenzfläche freigegeben oder aufgenommen wird. Insbesondere kann die Thermokette, im Speziellen deren erster oder zweiter Bereich, weniger als 1 µm, 20 µm, 1 mm, 5 mm, 1 cm oder weniger als 2 cm von der Grenzfläche beabstandet sein.

Gemäß einer Variante (Typ A) kann die Thermokette derart an der Grenzfläche bereitgestellt werden, dass ihr erster Bereich innerhalb der ersten Phase vorliegt und ihr zweiter Bereich innerhalb der zweiten Phase vorliegt. Dies ist insbesondere zum Vermessen eines flüssig-gasförmig Übergangs vorteilhaft, da hier der erste Bereich der Thermokette einfach in die flüssige Phase eingetaucht werden kann. Beispielsweise kann so die Verdunstungsrate von einer Wasseroberfläche bestimmt werden.

Gemäß einer anderen Variante (Typ B) wird die Thermokette vollständig auf der der ersten Phase oder der der zweiten Phase entsprechenden Seite der Grenzfläche angeordnet. Die Thermokette befindet sich also vollständig innerhalb der ersten oder der zweiten Phase. Gemäß dieser Ausführungsform wird der erste Bereich der Thermokette näher an der Grenzfläche positioniert als der zweite Bereich der Thermokette, so dass die aufgrund der latenten Wärme senkrecht zur Grenzfläche vorhandene Temperaturdifferenz über die Thermospannung der Thermokette messbar ist. Eine solche Anordnung ist besonders von Vorteil, wenn sich eine Phase in einer schwer zugänglichen Region befindet, wie z.B. Wasser (flüssige Phase) in einer Gebäudewand. Dann kann die Thermokette außerhalb der schwer zugänglichen Region angeordnet werden, beispielsweise in der Gasphase außerhalb der Gebäudewand. Es könnte beispielsweise über einen Feuchte- und Temperatursensor das Wasserpotential außerhalb der Gebäudewand bestimmt werden und dann unter Berücksichtigung der gemessenen Thermospannung einer an der Gebäudewand positionierten oder angebrachten Thermokette mittels des Darcy-Gesetzes das Wasserpotential der Gebäudewand bestimmt werden.

Gemäß einer weiteren Variante (Typ C) wird die Thermokette so an der Grenzfläche bereitgestellt, dass ihr erster Bereich und ihr zweiter Bereich in einer zu der Grenzfläche parallelen, gemeinsamen Ebene liegen. An sich wäre damit keine Temperaturdifferenz aufgrund der latenten Wärme des Phasenübergangs detektierbar, da beide Bereiche der Thermokette in gleichem Abstand zu der Grenzfläche befindlich sind. Um dennoch eine Messung zu ermöglichen, wird erfindungsgemäß die Grenzfläche am ersten Bereich der Thermokette mit einer Abdeckung abgedeckt, nicht aber am zweiten Bereich der Thermokette. Die Abdeckung kann zwischen dem ersten Bereich der Thermokette und der Grenzfläche oder auf der der Grenzfläche gegenüber liegenden Seite des ersten Bereichs der Thermokette positioniert werden. Durch die Abdeckung wird ein Übergang zwischen der ersten und der zweiten Phase des Materials an dem ersten Bereich der Thermokette unterdrückt oder unterbunden. Dort wird daher keine oder eine geringere Menge latenter Wärme abgegeben oder entnommen. Am zweiten Bereich der Thermokette allerdings kann der Phasenübergang ungehindert stattfinden und latente Wärme abgegeben oder entzogen werden. Somit kann auch bei einer flach auf der Grenzfläche vorgesehenen Thermokette durch Messen der Thermospannung eine Übergangsrate zwischen den Phasen gemessen werden. Da gemäß dieser Ausführungsform der erste und der zweite Bereich der Thermokette in einer zu der Grenzfläche parallelen gemeinsamen Ebene liegen, kann ein unabhängig von dem Phasenübergang senkrecht zur Grenzfläche vorliegender externer Temperaturgradient die Messung nicht verfälschen. Dies ist z.B. bei einer Messung an einer Bodenoberfläche vorteilhaft, da so der von einer möglicherweise durch Sonneneinstrahlung aufgewärmten Bodenoberfläche rührende vertikale Temperaturgradient die Messung nicht beeinträchtigt.

Bei einigen Anwendungen ist bereits in dem zu vermessenden System eine "natürliche" Grenzfläche zwischen der festen oder flüssigen ersten Phase und der gasförmigen zweiten Phase vorhanden und das oben beschriebene Messverfahren könnte ohne Weiteres durchgeführt werden. Beispiele dafür sind das Vermessen der Verdunstung an einer Wasseroberfläche und das Vermessen der Transpiration eines Lebewesens.

Eine Vorrichtung gemäß eines nicht beanspruchten Beispiels erlaubt eine besonders leicht durchzuführende Messung der Übergangsrate an einer natürlichen Grenzfläche zwischen einer ersten, festen oder flüssigen Phase und einer zweite, gasförmigen Phase. Die Vorrichtung umfasst eine Haftschicht mit einer Unterseite und einer Oberseite, wobei die Unterseite an einer Grenzfläche zwischen der ersten und der zweiten Phase des Materials anbringbar ist. Bei der Grenzfläche kann es sich beispielsweise um die Haut eine Benutzers handeln, dessen Transpirationsrate gemessen werden soll. Auch das Anbringen an einer Pflanze oder Teilen einer Pflanze zum Bestimmen der Transpirationsrate derselben ist denkbar. Beispielsweise kann die Vorrichtung über die Unterseite der Haftschicht an eine transpirierende Unterseite eines Blattes angebracht werden. Zum Durchführen der Messung ist an der Haftschicht die Thermokette mit der Vielzahl von Leiterübergängen angebracht, wobei die Thermokette einen jeden zweiten der Leiterübergänge umfassenden ersten Bereich und einen die übrigen Leiterübergänge umfassenden zweiten Bereich aufweist und dazu ausgelegt ist, eine messbare Thermospannung auszugeben, die eine Temperaturdifferenz zwischen dem ersten und dem zweiten Bereich der Thermokette repräsentiert.

Besonders vorteilhaft kann eine solche Vorrichtung zum Durchführen von Messungen des Typs C sein. Dazu können der erste Bereich und der zweite Bereich der Thermokette in einer zu der Oberseite der Haftschicht parallelen gemeinsamen Ebene liegen, wobei eine Abdeckung vorgesehen ist, die die Haftschicht am ersten Bereich der Thermokette abdeckt, nicht aber am zweiten Bereich der Thermokette. Es sind aber auch andere Anordnungen der Thermokette an der Haftschicht denkbar, beispielsweise zum Durchführen von Messungen der Typen A und B.

Eine Vorrichtung gemäß eines weiteren nicht beanspruchten Beispiels ist besonders gut zum Messen einer Transpirationsrate eines Pflanzenblattes geeignet. Auch mit dieser Vorrichtung kann eine Übergangsrate zwischen einer ersten Phase eines Materials und einer zweiten Phase des Materials gemessen werden, wobei das Material in der ersten Phase fest oder flüssig und in der zweiten Phase gasförmig vorliegt. In der Anwendung auf das Pflanzenblatt liegt Wasser (Material) innerhalb des Blattes flüssig vor (erste Phase) und geht bei der Transpiration des Blattes an einer Blattunterseite (Grenzfläche) in den gasförmigen Zustand außerhalb des Blattes über (zweite Phase).

Die Vorrichtung gemäß dieses Beispiels umfasst die Thermokette mit einer Vielzahl von Leiterübergängen, wobei die Thermokette einen jeden zweiten der Leiterübergänge umfassenden ersten Bereich und einen die übrigen Leiterübergänge umfassenden zweiten Bereich aufweist und dazu ausgelegt ist, eine messbare Thermospannung auszugeben, die eine Temperaturdifferenz zwischen dem ersten und dem zweiten Bereich der Thermokette repräsentiert. Zwischen dem ersten Bereich und dem zweiten Bereich der Thermokette ist ein Schlitz zum Aufnehmen einer eine Grenzfläche zwischen der ersten Phase und der zweiten Phase umfassenden Probe vorhanden. Insbesondere kann in dem Schlitz ein Pflanzenblatt aufgenommen werden. Der erste Bereich der Thermokette befindet sich dann angrenzend an die Blattunterseite und der zweite Bereich der Thermokette angrenzend an die Blattoberseite. Da Pflanzenblätter typischerweise nur auf einer Seite transpirieren (für gewöhnlich an der Unterseite), kann über die Thermospannung an der Thermokette eine Temperaturdifferenz zwischen der Blattoberseite und der Blattunterseite gemessen werden, der durch die latente Wärme des Phasenübergangs durch die Transpiration an nur einer Blattseite entsteht. Damit kann die Transpirationsrate bestimmt werden.

Die Erfindung stellt eine Möglichkeit zur Verfügung, Messungen auch an Systemen durchzuführen, in denen zunächst keine Grenze zwischen einer festen oder flüssigen Phase und einer gasförmigen Phase vorhanden ist. Um in Systemen, in denen das Material in zwei Gebieten jeweils gasförmig vorliegt, eine Übergangsrate zwischen den Gebieten messen zu können (z.B. zur Bestimmung eines Druckpotentials), wird die erste Phase, insbesondere als flüssige Phase, künstlich erzeugt. Dies wird durch Bereitstellen einer porösen Substratplatte realisiert. Es ist ein bekanntes Phänomen, dass innerhalb eines porösen Substrats gasförmiges Material aufgrund von Effekten der Oberflächenspannung leichter kondensiert. Dieser Effekt skaliert mit dem Durchmesser der Poren des Substrats.

Der mittlere Porendurchmesser des Substrats ist vorteilhafterweise größer als der Durchmesser eines Moleküls des zu messenden Materials, aber kleiner als 100 µm. Um beispielsweise Wasserdampf zum Kondensieren zu bringen, kann ein poröses Substrat mit einem mittleren Porendurchmesser im Bereich von mehreren Nanometern bis einigen Mikrometern bereitgestellt werden. Mittlere Porendurchmesser von weniger als 100 µm, weniger als 50 µm, weniger als 10 µm, weniger als 1 µm oder weniger als 800 nm oder ein mittlerer Porendurchmesser zwischen 200 nm und 800 nm, zwischen 500 nm und 2 µm oder zwischen 200 nm und 5 µm sind besonders vorteilhaft.

Die poröse Substratplatte trennt ein erstes Gebiet, in dem das Material gasförmig vorliegt und ein zweites Gebiet, in dem das Material gasförmig vorliegt. Die Substratplatte sollte natürlich für das Material durchlässig sein. Dies kann dadurch erreicht werden, dass die Substratplatte von ihrer Oberseite zu ihrer Unterseite durchgängig miteinander verbundene Poren aufweist, durch die das Material passieren kann. Alternativ können die Poren beispielsweise nur an der Unterseite und/oder der Oberseite der Substratplatte, insbesondere als offene Poren, vorgesehen sein. In diesem Fall kann die Substratplatte aus einem für das Material passierbaren Werkstoff bestehen oder diesen umfassen.

Um von einem Gebiet in das andere Gebiet zu diffundieren, muss das Material die Substratplatte passieren. Dabei kondensiert das Material innerhalb der Substratplatte, so dass dort künstlich eine flüssige Phase erzeugt wird. Mittels einer Thermokette kann dann, analog zu dem oben beschriebenen Verfahren, die Übergangsrate aus dieser flüssigen Phase (erste Phase) in die Gasphase (zweite Phase) eines Gebiets bestimmt werden. Diese Übergangsrate erlaubt ein Bestimmen der Übergangsrate zwischen den beiden Gebieten. Im Wesentlichen wird also auch hier eine Übergangrate zwischen einer (künstlich erzeugten) ersten, festen oder flüssigen Phase und einer zweiten, gasförmigen Phase eines Materials gemessen.

Besonders vorteilhaft ist es, wenn die Thermokette bereits fest in die künstliche Grenzfläche integriert, beziehungsweise an dieser befestigt ist. In diesem Fall ist die Orientierung und Positionierung der Thermokette in Bezug auf die Grenzfläche über mehrere Messungen hinweg konstant. Da auch die Permeabilitätseigenschaften der Grenzfläche konstant (und überdies durch Auswahl einer geeigneten porösen Substratplatte einstellbar) sind, können einmalig durchgeführte Kalibrierungsmessungen und daraus erhaltene Referenzdaten auf einfache Weise für mehrere Messungen verwendet werden.

Eine erfindungsgemäße Vorrichtung zum Messen der Übergangsrate zwischen einer ersten Phase eines Materials und einer zweiten Phase des Materials stellt eine solche poröse künstliche Grenzfläche mit einer daran angebrachten Thermokette bereit. Auch bezüglich der Beschreibung der Vorrichtung soll das Material in der ersten Phase fest oder flüssig und in der zweiten Phase gasförmig vorliegen.

Es erschließt sich, dass die erfindungsgemäße Vorrichtung zum Durchführen des erfindungsgemäßen Verfahrens geeignet ist. Bezüglich des Verfahrens beschriebene Merkmale können selbstverständlich auf die Vorrichtung übertragen werden und umgekehrt.

Die Vorrichtung zum Messen der Übergangsrate umfasst eine poröse Substratplatte mit einer Unterseite und einer Oberseite. Vorteilhafte Eigenschaften bezüglich der Porengröße wurden bereits beschrieben.

An der porösen Substratplatte ist die Thermokette mit der Vielzahl von Leiterübergängen angebracht. Wie oben dargelegt, umfasst die Thermokette einen jeden zweiten der Leiterübergänge umfassenden ersten Bereich und einen die übrigen Leiterübergänge umfassenden zweiten Bereich und ist dazu ausgelegt, eine messbare Thermospannung auszugeben, die die Temperaturdifferenz zwischen dem ersten und dem zweiten Bereich repräsentiert.

Auch wenn die erste Phase künstlich erzeugt wird, sind mit allen oben beschriebenen Varianten der Positionierung der Thermokette (Typ A, Typ B und Typ C) Messungen möglich. Dabei zu beachten, dass die Grenzfläche zwischen der flüssigen, ersten Phase in den Poren und der gasförmigen, zweiten Phase bei der Verwendung der porösen Substratplatte keine zusammenhängende Grenzfläche mehr bilden muss. Die oben erläuterten Prinzipien der Positionierung der Thermokette in Bezug auf die Grenzfläche lassen sich aber wie folgt auf eine Positionierung der Thermokette in Bezug auf die poröse Substratplatte übertragen, da die Grenzfläche näherungsweise als innerhalb der Substratplatte parallel zu deren Unterseite und/oder Oberseite verlaufend angehsehen werden kann. Um Messungen vom Typ A durchführen zu können, kann gemäß eines nicht als Vorrichtung beanspruchten Beispiels der erste Bereich der Thermokette auf der Unterseite der Substratplatte und der zweite Bereich der Thermokette auf der Oberseite der Substratplatte vorgesehen sein (Vorrichtung Typ A).

Um Messungen vom Typ B durchführen zu können, kann die Thermokette gemäß einer Ausführungsform vollständig auf der Unterseite oder vollständig auf der Oberseite der Substratplatte befindlich sein, wobei der erste Bereich der Thermokette näher an der entsprechenden Seite der Substratplatte befindlich ist als der zweite Bereich der Thermokette (Vorrichtung Typ B). Insbesondere kann der erste Bereich der Thermokette direkt an der porösen Substratplatte befestigt sein.

Um Messung vom Typ C durchführen zu können, können gemäß einer alternativen Ausführungsform der erste und der zweite Bereich der Thermokette in einer zu der Unterseite und/oder der Oberseite der Substratplatte parallelen gemeinsamen Ebene liegen (Vorrichtung Typ C). In diesem Fall umfasst die Vorrichtung auch eine Abdeckung, die die poröse Substratplatte am ersten Bereich der Thermokette abdeckt, nicht aber am zweiten Bereich der Thermokette. Vorteilhafterweise ist die Abdeckung zwischen der Oberseite oder der Unterseite der Substratplatte und dem ersten Bereich der Thermokette vorgesehen. Die Abdeckung kann aber auch auf der der Substratplatte gegenüberliegenden Seite der Thermokette vorgesehen sein. Insbesondere kann die Abdeckung an der Substratplatte und/oder der Thermokette befestigt sein. Die Thermokette kann flach an der entsprechenden Seite der Substratplatte angebracht sein.

Um eine definierte Messumgebung zu schaffen, kann die Substratplatte mit der Thermokette (Typ A, Typ B oder Typ C) derart in einem Rohr vorgesehen oder anbringbar sein, dass sie das Rohr in zwei axial aufeinanderfolgende Abschnitte unterteilt. Eine solche Anordnung erlaubt ohne besonderen Aufwand eine Messung des Wasserpotentials eines Bodens durchzuführen. Das Rohr wird für die Messung einfach bis zu der Substratplatte in dem Boden versenkt. In dem von dem versenkten, unteren Abschnitt des Rohrs vorliegenden Bodenabschnitt liegt das Wasser typischerweise gasförmig vor. In dem aus dem Boden ragenden, oberen Abschnitt des Rohrs liegt Wasser in Form von Luftfeuchtigkeit ebenfalls gasförmig vor. Wie oben beschrieben, entsteht aufgrund der porösen Struktur in der porösen Substratplatte eine Phase flüssigen Wassers. Durch Messung der Thermospannung an der Thermokette kann daher, wie ebenfalls oben beschrieben, die Übergangsrate von Wasser zwischen dem unteren Abschnitt des Rohrs (Boden) und dem oberen Abschnitt des Rohrs (Luft) gemessen werden (indirekt über die Übertrittsrate aus der oder in die künstlich erzeugte flüssige Phase). Ersichtlicherweise ist dies ein Beispiel für eine Messung einer Übertrittsrate von Wasser zwischen zwei Gebieten, in denen Wasser gasförmig vorliegt. Über eine Temperatur- und Feuchtemessung im aus dem Boden ragenden Teil des Rohrs kann, wie oben beschrieben, das Wasserpotential der oberen Gasphase bestimmt werden. Hierzu kann an dem Rohr ein Feuchte- und Temperatursensor vorgesehen sein. Mithilfe des Darcy-Gesetzes ist dann das Wasserpotential des Bodens bestimmbar.

Zur Durchführung anderer Messungen kann die Vorrichtung alternativ zu dem Rohr ein Gefäß mit einem Boden umfassen. Die Substratplatte mit der Thermokette (Typ A, Typ B oder Typ C) kann derart in dem Gefäß vorgesehen oder anbringbar sein, dass sie das Aufnahmevolumen des Gefäßes in einen unteren, den Boden des Gefäßes umfassenden Teil und in einen oberen, insbesondere offenen Teil unterteilt. Der untere Teil des Gefäßes kann zur Aufnahme einer Probe ausgelegt sein, deren Wasserpotential bestimmt werden soll. Nach einer gewissen Zeit stellt sich ein Gleichgewicht ein und das Wasserpotential der Probe entspricht dem Wasserpotential der Gasphase in dem unteren Teil des Gefäßes. Liegt in dem oberen Teil des Gefäßes ebenfalls eine Gasphase von Wasser vor (z.B. in Luft), kann analog zu der Messung des Wasserpotentials des Erdbodens mit dem Rohr das Wasserpotential der Probe bestimmt werden. Dazu kann mithilfe eines Feuchte- und Temperaturmessers das Wasserpotential der Gasphase im oberen Teil des Gefäßes bestimmt werden.

Es ist aber auch möglich, mit der erfindungsgemäßen Vorrichtung mit Substratplatte eine Messung einer Übergangsrate zwischen einer bereits vorliegenden, flüssigen oder festen ersten Phase und einer gasförmigen zweiten Phase durchzuführen. Ein vorteilhaftes Beispiel einer solchen Messung ist eine Abwandlung der beschriebenen Messung des Wasserpotentials der sich in dem unteren Teil des Gefäßes befindlichen Probe. Hierzu wird in dem oberen Teil des Gefäßes eine Wasserschicht eingebracht. Die Substratplatte trennt die im unteren Teil des Gefäßes vorliegende Gasphase (zweite Phase) von der im oberen Teil vorliegenden Wasserschicht (erste Phase). In einer solchen Situation kann die Grenzfläche zwischen den Phasen als parallel zur Substratplatte angesehen werden. Zudem kann die Grenzfläche als innerhalb der Substratplatte oder an deren Oberfläche liegend angesehen werden. Über eine Messung der Thermospannung der an der Substratplatte vorgesehenen Thermokette kann dann die Übergangsrate zwischen den Phasen bestimmt werden. Ein Vorteil in dem Vorsehen der Wasserschicht im oberen Teil des Gefäßes liegt darin, dass das Wasserpotential im oberen Teil des Gefäßes auf einen bekannten Wert, insbesondere auf null, gesetzt wird. Damit muss kein Feuchte- und Temperaturmesser vorgesehen werden, wenn das Wasserpotential der Probe bestimmt werden soll.

Die vorliegende Erfindung betrifft auch die Verwendung des beschriebenen Verfahrens oder der beschriebenen Vorrichtung zum Messen einer Transpirationsrate eines Lebewesens, z.B. in bestimmten Belastungssituationen wie beim Sport, zum Messen einer Verdunstungsrate eines Bodens oder zum Bestimmen eines Wasserpotentials oder einer Permeabilität einer Materialzusammensetzung, insbesondere einer Folie und/oder eines Verpackungsmaterials.

Nachfolgend soll die Erfindung anhand von Ausführungsbeispielen mit Hilfe der beigefügten Zeichnungen näher erläutert werden. Dabei zeigen:
- Figur 1A: eine schematische Schnittansicht einer Messung des Typs A gemäß eines Beispiels zur Erläuterung des Messprinzips, wobei die Schnittebene senkrecht zu der Grenzfläche zwischen den Phasen steht;
- Figur 1B: eine schematische Schnittansicht einer Messung des Typs B gemäß eines Beispiels zur Erläuterung des Messprinzips, wobei die Schnittebene senkrecht zu der Grenzfläche zwischen den Phasen steht;
- Figur 1C: eine schematische Darstellung einer Messung des Typs C gemäß eines Beispiels zur Erläuterung des Messprinzips in Draufsicht auf die Grenzfläche zwischen den Phasen;
- Figur 2A: eine schematische Perspektivansicht einer Vorrichtung gemäß einer nicht als Vorrichtung beanspruchten Variante des Typs A;
- Figur 2B: eine schematische Perspektivansicht einer Vorrichtung gemäß einer Ausführungsform des Typs B;
- Figur 2C: eine schematische Perspektivansicht einer Vorrichtung gemäß einer Ausführungsform des Typs C;
- Figur 3A: eine schematische Darstellungen einer Messung gemäß eines nicht beanspruchten Beispiels, die das Bestimmen einer Permeabilität einer Trennschicht erlaubt;
- Figur 3B: eine schematische Darstellung einer Vorrichtung mit einer Haftschicht gemäß eines nicht beanspruchten Beispiels;
- Figur 3C: eine schematische Darstellung einer Vorrichtung , gemäß eines nicht beanspruchten Beispiels die besonders zum Messen der Transpirationsrate eines Pflanzenblattes geeignet ist;
- Figur 3D: eine Anwendung gemäß eines nicht beanspruchten Beispiels zum Messen des Wasserpotentials in einem Baumstamm;
- Figur 3E: eine schematische Darstellung einer Vorrichtung gemäß einer Ausführungsform, die besonders zur Bestimmung des Wasserpotentials einer Probe geeignet ist; und
- Figur 3F: eine schematische Darstellung einer Vorrichtung gemäß einer Ausführungsform, die besonders zur Bestimmung des Wasserpotentials eines Bodens geeignet ist.

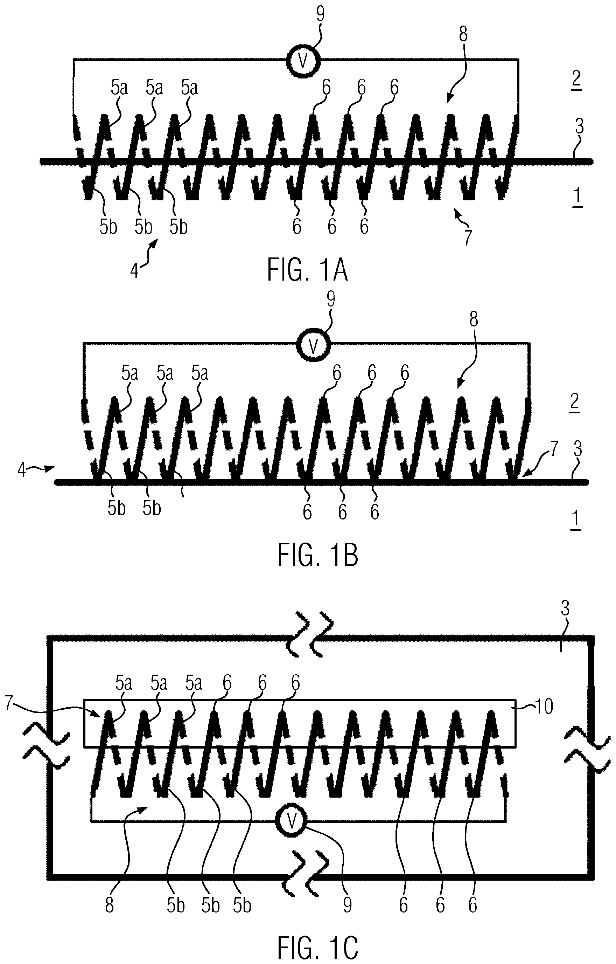

Die Figuren 1A, 1B und 1C illustrieren schematisch verschiedene Varianten des von der Erfindung verwendeten Messprinzips. Es wird jeweils eine Übergangsrate zwischen einer ersten Phase 1 und einer zweiten Phase 2 eines Materials gemessen. In der ersten Phase 1 liegt das Material fest oder flüssig vor und in der zweiten Phase 2 gasförmig. Das Material geht also durch Verdampfen/Kondensation oder durch Sublimation/Resublimation zwischen den Phasen 1, 2 über. Bei einem Übergang von der ersten Phase 1 zu der zweiten Phase 2 wird an einer Grenzfläche 3 zwischen den Phasen 1, 2 latente Wärme frei oder der Umgebung entzogen. Dadurch entsteht im Bereich der Grenzfläche 3 ein Temperaturgradient, insbesondere ein senkrecht zu der Grenzfläche 3 stehender Temperaturgradient. Die Erfindung nutzt aus, dass dieser Tempergradient ein Maß für die Übergangsrate zwischen den Phasen 1, 2 des Materials ist und ein Bestimmen der Übergangsrate erlaubt.

Die aufgrund der latenten Wärme im Bereich der Grenzfläche 3 vorliegende Temperaturdifferenz wird erfindungsgemäß mit einer Thermokette 4 bestimmt. Die Thermokette 4 umfasst eine Vielzahl von Leiterelementen 5a, 5b, wobei jeweils Leiterelemente 5a aus einem ersten Material, beispielsweise Konstantan oder Tellur, abwechselnd mit Leiterelementen 5b aus einem zweiten Material, beispielsweise Kupfer oder Antimon, hintereinander verbunden sind. Die Verbindungsstellen zwischen zwei aufeinanderfolgenden Leiterelementen 5a, 5b werden als Leiterübergänge 6 bezeichnet. Die Thermokette 4 weist einen jeden zweiten der Leiterübergänge 6 umfassenden ersten Bereich 7 und einen die übrigen Leiterübergänge 6 umfassenden zweiten Bereich 8 auf. Mittels einer Spannungsmessvorrichtung 9 wird eine zwischen den Enden der Thermokette 5 anliegende Thermospannung gemessen. Diese Thermospannung repräsentiert eine Temperaturdifferenz zwischen dem ersten 7 und dem zweiten Bereich 8 der Thermokette 4.

Durch geeignete Positionierung der Thermokette 4 an der Grenzfläche 3 zwischen den Phasen 1, 2 lässt sich der aufgrund der latenten Wärme vorhandene Temperaturgradient über die Thermospannung der Thermokette 4 messen und damit die Übergangsrate zwischen den Phasen 1, 2 bestimmen. In den Figuren sind aus Gründen der Übersichtlichkeit Thermoketten 4 mit einer relativ geringen Anzahl von Leiterübergängen 6 dargestellt. In der Praxis kann es vorteilhaft oder notwendig sein, wenn die Thermokette 4 eine deutlich größere Anzahl von Leiterübergängen 6 (z.B. zumindest 10, 50, 100, 500, 1000 oder mehr als 1000) umfasst, da so auch sehr kleine Temperaturdifferenzen gemessen werden können. Es erschließt sich ebenfalls, dass die in der vorliegenden Erfindung verwendete Thermokette 4 aus einer Mehrzahl über Verbindungselemente hintereinandergeschalteter Thermoketten 4 zusammengesetzt sein kann.

Zur geeigneten Positionierung der Thermokette 4 sind verschiedene Varianten denkbar.

Gemäß einer in Figur 1A gezeigten Variante (Typ A) wird die Thermokette 4 derart an der Grenzfläche 3 bereitgestellt, dass der erste Bereich 7 der Thermokette 4 innerhalb der ersten Phase 1 und der zweite Bereich 8 der Thermokette 4 innerhalb der zweiten Phase 2 vorliegt. Damit lässt sich ein zu der Grenzfläche 3 senkrecht stehender, von der latenten Wärme des Phasenübergangs stammender Temperaturgradient detektieren.

Eine andere Variante (Typ B) der Positionierung der Thermokette 4 an der Grenzfläche 3 ist in Figur 1B gezeigt. Hier wird die gesamte Thermokette 4 auf einer Seite der Grenzfläche 3 positioniert. In der gezeigten Ausführungsform ist die Thermokette 4 innerhalb der zweiten Phase 2 positioniert. Je nach den Eigenschaften der Phasen 1, 2 kann es aber auch vorteilhaft sein, die Thermokette 4 auf der anderen Seite der Grenzfläche 3 innerhalb der festen oder flüssigen ersten Phase 1 zu positionieren. Wie gezeigt, ist der erste Bereich 7 der Thermokette 4 näher an der Grenzfläche 3 als der zweite Bereich 8 der Thermokette 4. Vorzugsweise ist der erste Bereich 7 der Thermokette 4 direkt an oder auf der Grenzfläche 3 angebracht.

In den in den Figuren 1A und 1B gezeigten Varianten sind der erste 7 und der zweite Bereich 8 der Thermokette 4 zur Detektion der Temperaturdifferenz in einer senkrecht zu der Grenzfläche 3 stehenden Richtung voneinander beabstandet. Figur 1C zeigt in Draufsicht auf die Grenzfläche 3 schematisch eine alternative Variante (Typ C) der Positionierung der Thermokette 4 an der Grenzfläche 3, bei der dies nicht der Fall ist. Hier liegen der erste 7 und der zweite Bereich 8 der Thermokette 4 in einer zu der Grenzfläche 3 parallelen, gemeinsamen Ebene. Eine Messung der Übergangsrate zwischen den Phasen 1, 2 wird nun dadurch ermöglicht, dass am ersten Bereich 7 der Thermokette 4 der Phasenübergang unterdrückt wird. Dazu wird die Grenzfläche 3 in einem zu dem ersten Bereich 7 der Thermokette 4 korrespondierenden Bereich mit einer Abdeckung 10 abgedeckt. Vorzugsweise ist die Abdeckung 10 für das Material nicht oder nur geringfügig durchlässig.

In dem abgedeckten Bereich kann somit kein oder nur eine geringere Menge von Material zwischen der ersten Phase 1 und der zweiten Phase 2 übergehen. Daher ist in dem abgedeckten Bereich ein geringer oder kein Effekt der latenten Wärme vorhanden. Da allerdings an dem zweiten Bereich 8 der Thermokette 5 die Grenzfläche 3 nicht abgedeckt ist, ist dort ein Phasenübergang möglich. Am zweiten Bereich 8 der Thermokette 4 wird damit abhängig von der Übergangsrate des Phasenübergangs latente Wärme frei oder entnommen. Somit können auch in dieser Version durch Messung einer Temperaturdifferenz zwischen dem ersten 7 und dem zweiten Bereich 8 der Thermokette 4 Rückschlüsse über die Übergangsrate zwischen den Phasen 1, 2 gezogen werden. Besonders vorteilhaft ist, dass eine Messung des Typs C auch bei Vorliegen eines möglicherweise sogar variablen externen, senkrecht zu der Grenzfläche 3 stehenden Temperaturgefälles unverfälscht möglich ist.

Eine einfache Anwendung der in den Figuren 1A, 1B und 1C dargestellten Messverfahren ist beispielsweise das Bestimmen der Verdunstungsrate einer Flüssigkeit, wie z.B. Wasser oder Alkohol, unter bestimmten Bedingungen. Besonders einfach lässt sich eine solche Messung mit der in Figur 1A gezeigten Variante (Typ A) durchführen. Hier muss die Thermokette 4 lediglich mit ihrem ersten Bereich 7 in die Flüssigkeit (erste Phase 1) getaucht werden, wobei der zweite Bereich 8 aus der Flüssigkeit in die Gasphase (zweite Phase 2) ragt. Es ist aber auch möglich, Messungen der Varianten Typ B und Typ C zur Bestimmung der Verdampfungsgeschwindigkeit einer Flüssigkeit zu verwenden.

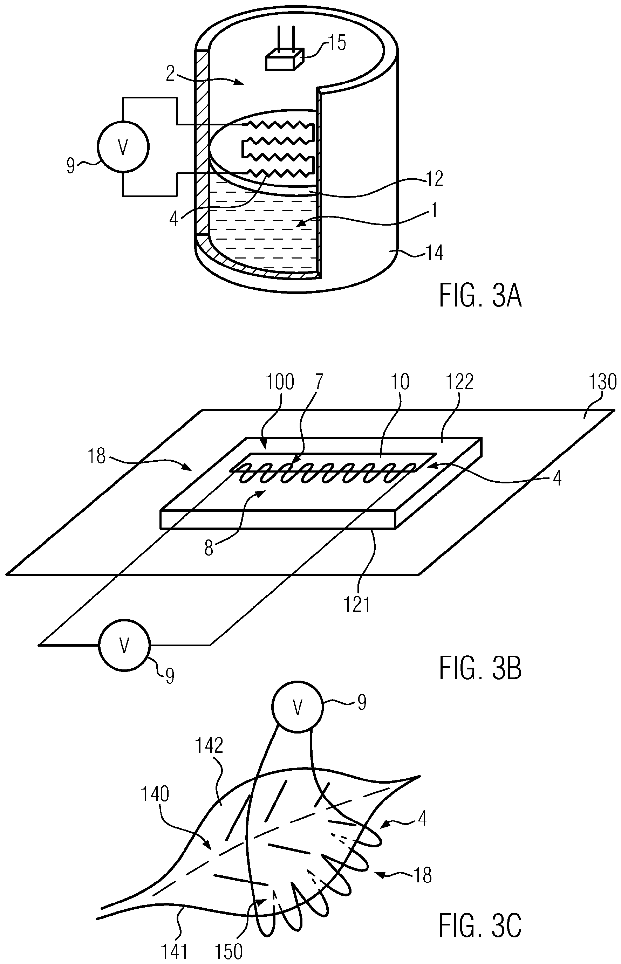

Figur 3A zeigt schematisch eine nicht beanspruchte spezielle Anwendung zum Bestimmen einer Permeabilität einer zu charakterisierenden Trennschicht 12. Bei der Trennschicht 12 kann es sich beispielsweise um eine Folie, insbesondere eine Folie mit Atmungsöffnungen, um Kunststoff oder eine Textilschicht handeln. Eine erste Phase 1 des Materials, bezüglich dessen die Permeabilität der Trennschicht 12 bestimmt werden soll, wird zur Messung in ein Gefäß 14 (zur besseren Übersichtlichkeit geschnitten dargestellt) gegeben und dann mit der Trennschicht 12 abgedeckt. Die Trennschicht 12 bildet nun die Grenzfläche 3 zwischen der ersten Phase 1 und einer über der Trennschicht 12 vorgesehenen Gasphase (zweite Phase 2) des Materials.

Für die Permeabilitätsbestimmung wird zunächst eine Messung der Übergangsrate zwischen der ersten Phase 1 und der zweiten Phase 2 des Materials mit dem beschriebenen Verfahren durchgeführt. Dazu wird die Thermokette 4 an der Trennschicht 12 bereitgestellt.

Dies kann gemäß den oben beschriebenen Varianten geschehen (Typ A, Typ B oder Typ C). Besonders geeignet ist hier eine Anordnung der Thermokette 4 gemäß den in den Figuren 1B und 1C dargestellten Varianten Typ B oder Typ C. Prinzipiell wäre auch die in Figur 1A gezeigte Variante denkbar. Hier ist allerdings zu beachten, dass dazu die Thermokette 4 die zu untersuchende Trennschicht 12 durchdringen muss und somit an den entsprechenden Durchdringungsstellen die zu messende Permeabilität der Trennschicht 12 verfälscht werden kann.

Basierend auf der gemessenen Thermospannung lässt sich die Permeabilität der Trennschicht 12 über das Darcy-Gesetz bestimmen. Wie oben beschrieben, lautet dieses Vt = k^{∗}(p2 - p1), wobei Vt die Übergangsrate von der ersten Phase 1 in die zweite Phase 2, p1 das Druckpotential der ersten Phase, p2 das Druckpotential der zweiten Phase und k ein die Permeabilität der Trennschicht 12 zwischen den Phasen 1, 2 repräsentierender Proportionalitätsfaktor ist. Die Übergangsrate Vt kann aus der gemessenen Thermospannung bestimmt werden. Zur Bestimmung des die Permeabilität der Trennschicht 12 repräsentierenden Proportionalitätsfaktors k sind zudem noch die Druckpotentiale p1, p2 der beiden Phasen erforderlich. Das Druckpotential p2 der Gasphase wird vorteilhafterweise, wie oben beschrieben, über eine Temperatur-und Feuchtemessung bestimmt. Dazu kann innerhalb der Gasphase ein Temperatur-und Feuchtesensor 15 vorgesehen sein, welcher beispielsweise an dem Gefäß 14 befestigt sein kann. Das Druckpotential p1 der ersten Phase 1 kann ebenfalls gemessen werden. Vorteilhafterweise ist dieses allerdings ohnehin bekannt, beispielsweise, wenn das Material in der ersten Phase 1 einfach als reine Flüssigkeit vorliegt.

Eine weitere nicht beanspruchte Anwendung und eine entsprechende Vorrichtung 18 sind in Figur 3B gezeigt. Die Vorrichtung 18 umfasst eine Haftschicht 100 mit einer Unterseite 121 und einer Oberseite 122. Mit der Unterseite 121 kann die Haftschicht 100 schnell und simpel an einer zu vermessenden Grenzfläche 3 zwischen einer ersten Phase 1 und einer zweiten Phase 2 angebracht werden kann. In dem gezeigten Beispiel ist die Unterseite 121 der Haftschicht 100 an der Hautoberfläche 130 eines Benutzers angebracht, um dessen Transpirationsrate zu messen. Eine entsprechende Vorrichtung 18 könnte beispielsweise beim Sport oder zu medizinischen Zwecken eingesetzt werden. Die Unterseite 121 der Haftschicht 110 könnte auch an einem Teil einer Pflanze, z.B. einem Blatt, angebracht werden. An der Haftschicht 100, insbesondere an deren Oberseite 122, ist die Thermokette 4 angebracht, so dass über die Thermospannung die Übergangsrate
zwischen den Phasen bestimmt werden kann. In der gezeigten Ausführungsform liegen der erste Bereich 7 und der zweite Bereich 8 der Thermokette 4 in einer zu der Oberseite 121 der Haftschicht 100 parallelen gemeinsamen Ebene. Zudem ist eine Abdeckung 10 vorgesehen, die die Haftschicht 100 am ersten Bereich 7 der Thermokette 4 abdeckt, nicht aber am zweiten Bereich 8 der Thermokette 4. Die gezeigte Vorrichtung 18 ist also für Messungen vom Typ C ausgelegt. Es wäre aber auch denkbar, die Thermokette 4 so an der Haftschicht 100 anzubringen, dass Messungen vom Typ A oder B möglich sind. Es ist denkbar, dass die Haftschicht 100 zur Erhöhung der Stabilität der Vorrichtung eine gewisse Dicke aufweist. Die Haftschicht 100 könnte aber auch lediglich als sehr dünne Klebeschicht ausgebildet sein, die ein Befestigen der Thermokette 4 erlaubt, die Durchlässigkeit der Grenzfläche 3 aber nicht wesentlich beeinträchtigt. Insbesondere ist es nicht notwendig, dass die Haftschicht 100 eine zusammenhängende Schicht darstellt. Es wäre denkbar, dass die Haftschicht nur bestimmte, befestigungsrelevante Bereiche der Thermokette 4 abdeckt.

Figur 3C zeigt eine weitere nicht beanspruchte Anwendung und eine entsprechende Vorrichtung 18. Die gezeigte Vorrichtung 18 ist besonders zur Messung einer Transpirationsrate eines Pflanzenblattes 140 geeignet. Gemäß dieser Ausführungsform umfasst die Vorrichtung 18 eine besonders ausgebildete Thermokette 4. Zwischen dem ersten Bereich 7 und dem zweiten Bereich 8 der Thermokette 4 ein Schlitz 150 zum Aufnehmen des Pflanzenblattes 140 vorgesehen. Der erste Bereich 7 der Thermokette 4 grenzt bei eingeschobenem Pflanzenblatt 140 an die Blattunterseite 141 und der zweite Bereich 8 der Thermokette 4 grenzt an die Blattoberseite 142. Da Pflanzenblätter 140 typischerweise nur auf einer Seite transpirieren (für gewöhnlich an der Unterseite 141), kann über die Thermospannung an der Thermokette 4 eine Temperaturdifferenz zwischen der Blattoberseite 142 und der Blattunterseite 141 gemessen werden, die durch die latente Wärme des Phasenübergangs durch die Transpiration an nur einer Blattseite 141 entsteht. Damit kann die Transpirationsrate des Blattes 140 bestimmt werden. Selbstverständlich können mit der Vorrichtung 18 auch andere Proben untersucht werden, die nur auf einer Seite Transpiration zeigen.

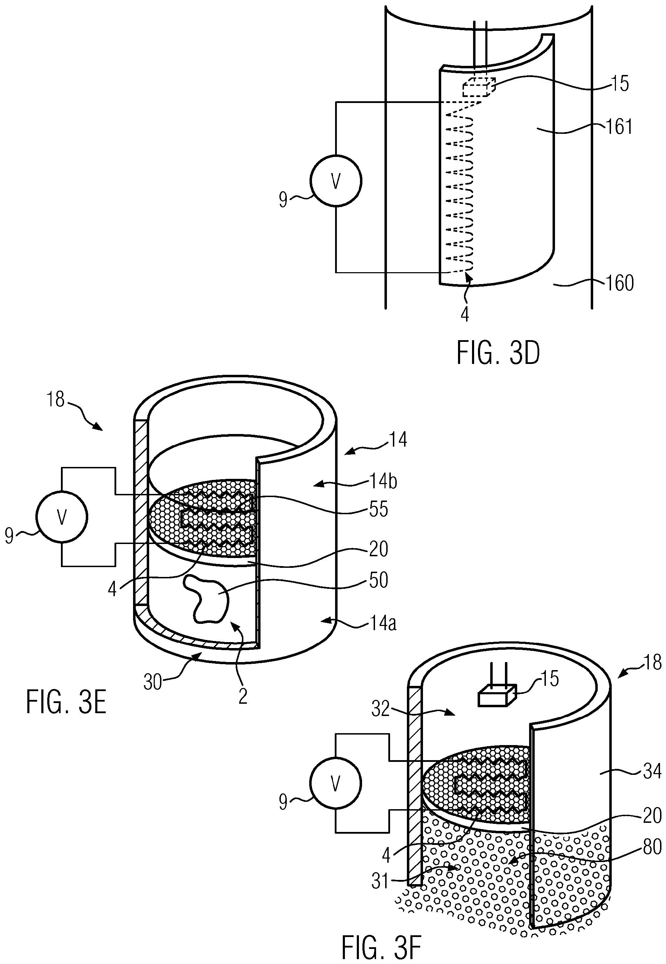

Figur 3D zeigt eine nicht beanspruchte Anwendung zum Messen des Wasserpotentials in einem Baumstamm. Eine Thermokette 4 des Typs C wird an der lebenden Rinde 160 des Baums angebracht. Mit einer Kappe 161 wird die Thermokette 4 gegen Regen und Witterungseinflüsse geschützt. Über die Thermospannung der Thermokette 4 kann die Übergangsrate von einer flüssigen Phase innerhalb des Baumstammes in eine außerhalb vorliegende Gasphase bestimmt werden. Wenn zudem innerhalb der Kappe 161 ein Feuchte-und Temperatursensor 15 vorgesehen ist, lässt sich zudem das Wasserpotential innerhalb des Baumstammes bestimmen.

In der Praxis kann es vorkommen, dass bei einem zu vermessenden System von sich aus keine Grenzfläche zwischen einer festen oder flüssigen Phase und einer gasförmigen Phase vorliegt, sondern lediglich eine Gasphase in einem ersten Gebiet 31 und eine Gasphase in einem zweiten Gebiet 32. In diesem Fall kann eine flüssige Phase 1 und somit eine Grenzfläche 3 zwischen dieser und einer der Gasphasen durch eine (offenporige) poröse Substratplatte 20 mit einer Unterseite 21 und einer Oberseite 22 künstlich erzeugt werden. Dieses Vorgehen basiert darauf, dass innerhalb der porösen Substratplatte 20 gasförmiges Material (Dampf) aufgrund von Effekten der Oberflächenspannung leichter kondensiert. Dieser Effekt skaliert mit dem Durchmesser der Poren 25 der Substratplatte 20. Die Substratplatte 20 sollte natürlich für das Material durchlässig sein. Dies kann dadurch erreicht werden, dass die Substratplatte 20 von ihrer Oberseite 22 zu ihrer Unterseite 21 durchgängig miteinander verbundene Poren 25 aufweist, durch die das Material passieren kann. Alternativ können die Poren 25 beispielsweise nur an der Unterseite 21 und/oder der Oberseite 22 der Substratplatte, insbesondere als offene Poren 25, vorgesehen sein. In diesem Fall kann die Substratplatte 20 aus einem für das Material passierbaren Werkstoff bestehen oder diesen umfassen.

Eine der beiden Seiten 21, 22 der Substratplatte 20 ist dem ersten Gebiet 31 zugewandt und die andere Seite 21, 22 ist dem zweiten Gebiet 32 zugewandt. Um zwischen dem ersten und dem zweiten Gebiet 31, 32 zu diffundieren, muss das Material daher die poröse Substratplatte 20 passieren. Dabei kondensiert das Material innerhalb der Substratplatte 20, so dass dort künstlich eine flüssige Phase 1 erzeugt wird. Mittels einer Thermokette 4 kann dann, analog zu dem oben beschriebenen Verfahren, die Übergangsrate aus dieser flüssigen Phase (erste Phase 1) in die Gasphase (zweite Phase 2) eines Gebiets 31, 32 bestimmt werden. Diese Übergangsrate erlaubt ein Bestimmen der Übergangsrate zwischen den beiden Gebieten 31, 32. Im Wesentlichen wird also auch hier eine Übergangrate zwischen einer (künstlich erzeugten) ersten, festen oder flüssigen Phase 1 und einer zweiten, gasförmigen Phase 2 eines Materials gemessen.

Die Übergangsrate zwischen den Phasen 1, 2 kann gemäß den oben beschriebenen Prinzipien mit einer an der Substratplatte 20 bereitgestellten Thermokette 4 gemessen werden. Es ist besonders vorteilhaft, wenn die Thermokette 4 bereits fest in die poröse Substratplatte 20 integriert, beziehungsweise an dieser befestigt ist, so dass die Orientierung und Positionierung der Thermokette 4 in Bezug auf die Substratplatte 20 fest definiert und konstant ist. Da auch die Permeabilitätseigenschaften der porösen Substratplatte 20 konstant sind, können einmalig durchgeführte Kalibrierungsmessungen und daraus erhaltene Referenzdaten auf einfache Weise für mehrere Messungen verwendet werden.

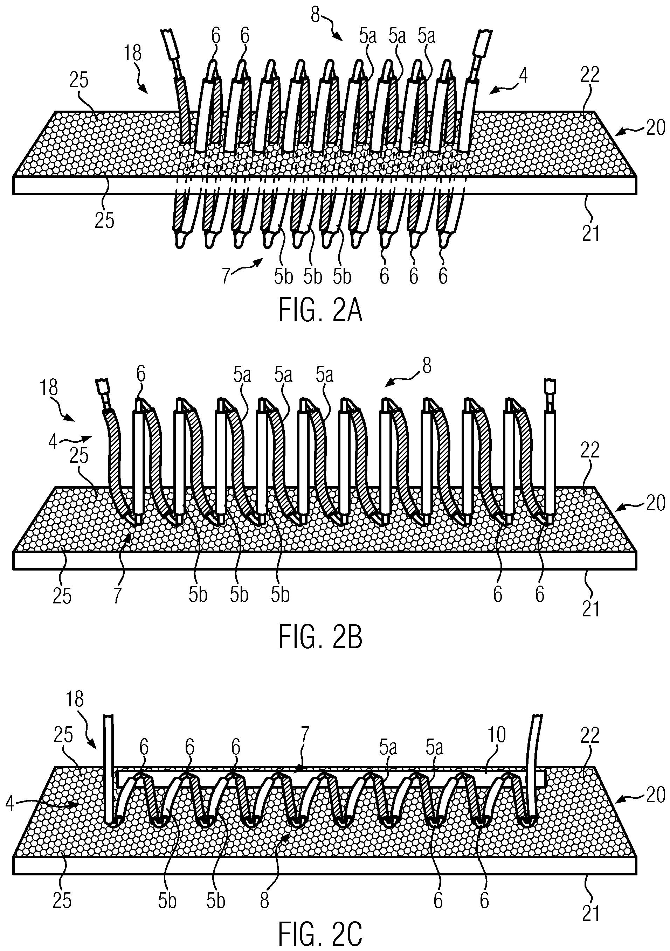

Die Figuren 2A, 2B und 2C zeigen verschiedene Varianten einer Vorrichtung 18 mit einer porösen Substratplatte 20 zum Erzeugen einer künstlichen Grenzfläche 3 und einer daran angebrachten Thermokette 4. Die Varianten unterscheiden sich in der Anordnung der Thermokette 4 in Bezug auf die poröse Substratplatte 20.

Um Messungen vom Typ A durchführen zu können, ist gemäß der in Figur 2A gezeigten Variante der Vorrichtung 18 der erste Bereich 7 der Thermokette 4 auf der Unterseite 21 der Substratplatte 20 und der zweite Bereich 8 der Thermokette 4 auf der Oberseite 22 der Substratplatte 20 vorgesehen (Vorrichtung Typ A). Diese Variante ist nicht als Vorrichtung beansprucht.

Um Messungen vom Typ B durchführen zu können, ist gemäß der in Figur 2B gezeigten erfindungsgemäßen Variante der Vorrichtung 18 (Vorrichtung Typ B) die Thermokette 4 vollständig auf der Oberseite 22 der Substratplatte 20 befindlich, wobei der erste Bereich 7 der Thermokette 4 näher an der Oberseite 22 der Substratplatte 20 befindlich ist als der zweite Bereich 8 der Thermokette 4. Alternativ kann Thermokette 4 vollständig auf der Unterseite 21 der Substratplatte 20 befindlich sein, wobei der erste Bereich 7 der Thermokette 4 näher an der Unterseite 21 der Substratplatte 20 befindlich ist als der zweite Bereich 8 der Thermokette 4. Insbesondere kann der erste Bereich 7 der Thermokette 4 direkt an der porösen Substratplatte 20 befestigt sein.

Um Messungen vom Typ C durchführen zu können, liegen gemäß der in Figur 2C gezeigten erfindungsgemäßen Variante der Vorrichtung 18 der erste 7 und der zweite Bereich 8 der Thermokette 4 in einer zu der Unterseite 21 und/oder der Oberseite 22 der Substratplatte 20 parallelen gemeinsamen Ebene (Vorrichtung Typ C). In der dargestellten Ausführungsform ist die Thermokette 4 flach an der Oberseite 22 der Substratplatte 20 angebracht. Alternativ kann die Thermokette 4 flach an der Unterseite 21 der Substratplatte 20 angebracht sein. Eine Vorrichtung 18 gemäß Typ C umfasst auch die oben beschriebene Abdeckung 10, die die poröse Substratplatte 20 am ersten Bereich 7 der Thermokette 4 abdeckt, nicht aber am zweiten Bereich 8 der Thermokette 4. In der gezeigten Ausführungsform ist die Abdeckung 10 zwischen der Substratplatte 20 und dem ersten Bereich 7 der Thermokette 4 vorgesehen.

Die Abdeckung 10 könnte aber auch auf der der Substratplatte 20 gegenüberliegenden Seite der Thermokette 4 vorgesehen sein. Insbesondere kann die Abdeckung 10 an der porösen Substratplatte 20 und/oder der Thermokette 4 befestigt sein.

Die Figuren 3E und 3F zeigen Ausführungsformen einer erfindungsgemäßen Vorrichtung 18, bei der die poröse Substratplatte 20 in eine definierte Messumgebung integriert ist.

Die in Figur 3F dargestellte Ausführungsform ist besonders gut zur Bestimmung des Wasserpotentials eines Bodens 80 geeignet. Die hier gezeigte Vorrichtung 18 umfasst ein Rohr 34 (zur besseren Übersichtlichkeit geschnitten dargestellt). Die poröse Substratplatte 20 mit der Thermokette 4 (Typ B oder Typ C) ist derart in dem Rohr 34 vorgesehen oder angebracht, dass sie das Rohr 34 in zwei axial aufeinanderfolgende Abschnitte (oberer und unterer Abschnitt) unterteilt. In dem oberen Abschnitt ist ein Temperatur-und Feuchtesensor 15 vorgesehen. Zur Messung wird das Rohr 34 einfach bis zu der Substratplatte 20 in dem zu vermessenden Boden 80 versenkt und dann die Thermospannung an der Thermokette 4 gemessen. In dem in dem versenkten, unteren Abschnitt des Rohrs 34 vorliegenden Bodenabschnitt liegt das Wasser typischerweise gasförmig vor (erstes Gebiet 31). In dem aus dem Boden 80 ragenden, oberen Abschnitt des Rohrs 34 liegt Wasser in Form von Luftfeuchtigkeit ebenfalls gasförmig vor (zweites Gebiet 32). Wie oben beschrieben, entsteht aufgrund der porösen Struktur in der porösen Substratplatte 20 eine Phase flüssigen Wassers. Durch Messung der Thermospannung an der Thermokette 4 kann daher, wie ebenfalls oben beschrieben, die Übergangsrate von Wasser zwischen dem ersten Gebiet 31 im unteren Abschnitt des Rohrs 34 (Boden 80) und dem zweiten Gebiet 32 im oberen Abschnitt des Rohrs 34 (Luft) gemessen werden (indirekt über die Übertrittsrate aus der oder in die künstlich erzeugte flüssige Phase). Ersichtlicherweise ist dies ein Beispiel für eine Messung einer Übertrittsrate von Wasser zwischen zwei Gebieten 31, 32, in denen Wasser gasförmig vorliegt. Über eine Temperatur- und Feuchtemessung im aus dem Boden 80 ragenden Teil des Rohrs 34 kann das Wasserpotential im zweiten Gebiet 32 (Luft über dem Boden 80) bestimmt werden. Damit ist dann unter Berücksichtigung der gemessenen Thermospannung eine Bestimmung des Wasserpotentials des Bodens 80 möglich.

Zur Durchführung anderer Messungen kann die Vorrichtung 18, wie in Figur 3E gezeigt, alternativ zu dem Rohr 34 ein Gefäß 14 (zur besseren Übersichtlichkeit geschnitten dargestellt) mit einem Boden 30 umfassen. Die Substratplatte 20 mit der Thermokette 4 (Typ B oder Typ C) kann derart in dem Gefäß 14 vorgesehen oder anbringbar sein, dass sie das Aufnahmevolumen des Gefäßes 14 in einen unteren, den Boden 30 des Gefäßes 14 umfassenden Teil 14a und in einen oberen, insbesondere offenen Teil 14b unterteilt. Der untere Teil 14a des Gefäßes 14 kann zur Aufnahme einer Probe 50 (Pflanzen, Boden, Medikamente, Lebensmittel etc.) ausgelegt sein, deren Wasserpotential bestimmt werden soll. Nach einer gewissen Zeit stellt sich ein Gleichgewicht ein und das Wasserpotential der Probe 50 entspricht dem Wasserpotential der Gasphase in dem unteren Teil 14a des Gefäßes 14. In den oberen Teil 14b des Gefäßes 14 wird eine Wasserschicht 55 eingebracht. Die Substratplatte 20 trennt die im unteren Teil 14a des Gefäßes 14 vorliegende Gasphase (zweite Phase 2) von der im oberen Teil vorliegenden Wasserschicht 55 (erste Phase 1). Über eine Messung der Thermospannung der an der Substratplatte 20 vorgesehenen Thermokette 4 kann dann die Übergangsrate zwischen den Phasen 1, 2 bestimmt werden. Diese Anwendung ist ein Beispiel für den Einsatz der porösen Substratplatte 20 ohne dass diese künstlich eine flüssige Phase erzeugt. Ein Vorteil in dem Vorsehen der Wasserschicht 55 im oberen Teil 14b des Gefäßes 14 liegt darin, dass das Wasserpotential im oberen Teil 14b des Gefäßes 14 auf einen bekannten Wert, insbesondere auf null, gesetzt wird. Damit muss kein Feuchte- und Temperaturmesser vorgesehen werden, wenn das Wasserpotential der Probe 50 bestimmt werden soll.

In den Figuren 3A, 3E und 3F ist die Thermokette 4 schematisch als Hintereinanderschaltung mehrerer kürzerer, hintereinandergeschalteter Thermoketten 4 dargestellt. Dies kann z.B. vorteilhaft sein, wenn eine poröse Substratplatte 20 einer bestimmten Form möglichst vollständig zur Messung genutzt werden soll. Für die gezeigten Anwendungen sind jeweils Anordnungen der Thermokette 4 gemäß allen vorgestellten Varianten (Typ A, Typ B oder Typ C) denkbar. Soll eine aus mehreren kürzeren Thermoketten 4 zusammengesetzte Thermokette 4 gemäß Typ C an der Grenzfläche 3 positioniert werden, erschließt sich, dass entsprechend den mehreren kürzeren Thermoketten 4 mehrere Abdeckungen 10 eingesetzt werden können.

## Patentansprüche

1. Vorrichtung (18) zum Messen einer Übergangsrate zwischen einer ersten Phase (1) eines Materials und einer zweiten Phase (2) des Materials, wobei das Material in der ersten Phase (1) fest oder flüssig und in der zweiten Phase (2) gasförmig vorliegt, umfassend:
eine poröse Substratplatte (20) zum künstlichen Erzeugen der ersten Phase (1), wobei die poröse Substratplatte (20) eine Unterseite (21) und eine Oberseite (22) aufweist; und
eine an der porösen Substratplatte (20) angebrachte Thermokette (4) mit einer Vielzahl von Leiterübergängen (6), wobei die Thermokette (4) einen jeden zweiten der Leiterübergänge (6) umfassenden ersten Bereich (7) und einen die übrigen Leiterübergänge (6) umfassenden zweiten Bereich (8) aufweist und dazu ausgelegt ist, eine messbare Thermospannung auszugeben, die eine Temperaturdifferenz zwischen dem ersten und dem zweiten Bereich (7, 8) der Thermokette (4) repräsentiert, wobei
entweder die Thermokette (4) vollständig auf der Unterseite (21) oder auf der Oberseite (22) der Substratplatte (20) gelegen ist und der erste Bereich (7) der Thermokette (4) näher an der entsprechenden Seite (12, 22) der Substratplatte (20) befindlich ist als der zweite Bereich (8) der Thermokette (4),
oder der erste Bereich (7) und der zweite Bereich (8) der Thermokette (4) in einer zu der Unterseite (21) und/oder der Oberseite (22) der Substratplatte (20) parallelen gemeinsamen Ebene liegen und eine Abdeckung (10) vorgesehen ist, die die poröse Substratplatte (20) am ersten Bereich (7) der Thermokette (4) abdeckt, nicht aber am zweiten Bereich (8) der Thermokette (4).

2. Vorrichtung nach Anspruch 1, die zudem ein Gefäß (14) mit einem Boden (30) umfasst, wobei die poröse Substratplatte (20) derart in dem Gefäß (14) anbringbar ist, dass sie das Aufnahmevolumen des Gefäßes (14) in einen unteren, den Boden (30) umfassenden Teil (14a) und einen oberen Teil (14b) unterteilt.

3. Vorrichtung nach Anspruch 1, die zudem ein Rohr (34) umfasst, wobei die poröse Substratplatte (20) derart in dem Rohr (34) angebracht ist, dass sie das Rohr (34) in zwei axial aufeinanderfolgende Abschnitte unterteilt.

4. Vorrichtung nach einem der vorangehenden Ansprüche, die zudem einen Feuchte- und Temperatursensor (15) umfasst.

5. Vorrichtung nach einem der vorangehenden Ansprüche, wobei die poröse Substratplatte (20) einen mittleren Porendurchmesser von weniger als 100 µm, weniger als 50 µm, weniger als 10 µm, weniger als 1 µm oder weniger als 800 nm oder einen mittleren Porendurchmesser zwischen 200 nm und 800 nm, zwischen 500 nm und 2 µm oder zwischen 200 nm und 5 µm aufweist.

6. Verfahren zum Messen einer Übergangsrate zwischen einer ersten Phase (1) eines Materials und einer zweiten Phase (2) des Materials, wobei das Material in der ersten Phase (1) fest oder flüssig und in der zweiten Phase (2) gasförmig vorliegt, umfassend:
künstliches Erzeugen der ersten Phase (1) durch eine poröse Substratplatte (20);
Bereitstellen einer eine Vielzahl von Leiterübergängen (6) umfassenden Thermokette (4) an einer Grenzfläche (3) zwischen der ersten und der zweiten Phase (1, 2), wobei die Thermokette (4) einen jeden zweiten der Leiterübergänge (6) umfassenden ersten Bereich (7) und einen die übrigen Leiterübergänge (6) umfassenden zweiten Bereich (8) aufweist; und
Messen einer an der Thermokette (4) anliegenden Thermospannung, die eine Temperaturdifferenz zwischen dem ersten und dem zweiten Bereich (7, 8) der Thermokette (4) repräsentiert.

7. Verfahren gemäß Anspruch 6, wobei die Thermokette (4) fest in die Substratplatte (20) integriert oder an dieser befestigt ist.

8. Verfahren zum Bestimmen eines Druckpotentials umfassend:
Durchführen des Verfahrens nach Anspruch 6 oder 7 und
Bestimmen des Druckpotentials der ersten Phase (1) des Materials oder der zweiten Phase des Materials (2) basierend auf einem bekannten oder gemessenen Wert des Druckpotentials der anderen Phase (1, 2) des Materials und der gemessenen Thermospannung.

9. Verfahren zum Bestimmen einer Permeabilität einer Trennschicht (12), insbesondere einer Folie oder einer Textilschicht, umfassend:
Durchführen des Verfahrens nach Anspruch 6 oder 7, wobei die Trennschicht (12) dabei zwischen der ersten Phase (1) und der zweiten Phase (2) vorliegt;
Bestimmen der Permeabilität der Trennschicht (12) anhand der gemessenen Thermospannung und bekannter oder gemessener Werte der Druckpotentiale der beiden Phasen (1, 2) des Materials.

10. Verfahren nach einem der Ansprüche 6-9, wobei das Verfahren zum Messen einer Transpirationsrate eines Lebewesens, zum Messen einer Verdunstungsrate eines Bodens (80) oder zum Bestimmen des Wasserpotentials oder der Permeabilität einer Materiezusammensetzung angewendet wird.

11. Verfahren nach Anspruch 10, wobei es sich bei der Materialzusammensetzung um eine Folie und/oder ein Verpackungsmaterial handelt.

## Claims

1. Device (18) for measuring a transition rate between a first phase (1) of a material and a second phase (2) of the material, wherein the material is solid or liquid in the first phase (1) and gaseous in the second phase (2), comprising:
a porous substrate plate (20) for artificially creating the first phase (1), wherein the porous substrate plate (20) comprises a lower side (21) and an upper side (22); and
a thermopile (4) installed at the porous substrate plate (20) and with a plurality of conductor transitions (6), wherein the thermopile (4) has a first portion (7), which comprises every second one of the conductor transitions (6), and a second portion (8), which comprises the remaining conductor transitions (6), and is designed to output a measurable thermoelectric voltage that represents a temperature difference between the first and the second portion (7, 8) of the thermopile (4), wherein
either the thermopile (4) is located completely on the lower side (21) or on the upper side (22) of the substrate plate (20) and the first portion (7) of the thermopile (4) is closer to the respective side (12, 22) of the substrate plate (20) than the second portion (8) of the thermopile (4),
or the first portion (7) and the second portion (8) of the thermopile (4) are located in a common plane that is parallel to the lower side (21) and/or the upper side (22) of the substrate plate (20) and a cover (10) is provided that covers the porous substrate plate (20) at the first portion (7) of the thermopile (4) but not at the second portion (8) of the thermopile (4).

2. Device according to Claim 1, further comprising a container (14) with a floor (30), wherein the porous substrate plate (20) can be installed in the container (14) in a way that it divides the receiving volume of the container (14) into a lower part (14a) that comprises the floor (30) and an upper part (14b).

3. Device according to Claim 1, further comprising a tube (34), wherein the porous substrate plate (20) is installed in the tube (34) in a way that it divides the tube (34) into two axially successive sections.

4. Device according to any one of the preceding Claims, further comprising a moisture and temperature sensor (15).

5. Device according to any one of the preceding Claims, wherein the porous substrate plate (20) has a mean pore diameter of less than 100 µm, less than 50 µm, less than 10 µm, less than 1 µm or less than 800 nm or a mean pore diameter between 200 nm and 800 nm, between 500 nm and 2 µm or between 200 nm and 5 µm.

6. Method for measuring a transition rate between a first phase (1) of a material and a second phase (2) of the material with the material being solid or liquid in the first phase (1) and gaseous in the second phase (2), comprising:
artificially creating the first phase (1) by a porous substrate plate (20);
providing a thermopile (4), which comprises a plurality of conductor transitions (6), at an interface area (3) between the first and the second phase (1, 2), wherein the thermopile (4) has a first portion (7), which comprises every second one of the conductor transitions (6), and a second portion (8) that comprises the remaining conductor transitions (6); and
measuring a thermoelectric voltage of the thermopile (4), which represents a temperature difference between the first and the second portion (7, 8) of the thermopile (4).

7. Method according to Claim 6, wherein the thermopile (4) is integrated firmly into the porous substrate plate (20) or attached to the porous substrate plate (20).

8. Method for determining a pressure potential, comprising:
carrying out the method according to Claim 6 or 7; and
determining the pressure potential of the first phase (1) of the material or the second phase of the material (2) based on a known or measured value of the pressure potential of the other phase (1, 2) of the material and the measured thermoelectric voltage.

9. Method for determining a permeability of a separation layer (12), in particular a plastic film or a textile layer, comprising:
carrying out the method according to Claim 6 or 7 while the separation layer (12) is located between the first phase (1) and the second phase (2);
determining the permeability of the separation layer (12) based on the measured thermoelectric voltage and known or measured values of the pressure potential of the two phases (1, 2) of the material.

10. Method according to any one of Claims 6 to 9, wherein the method is applied for measuring a transpiration rate of a living being, for measuring an evaporation rate of a ground (80) or for determining the water potential or the permeability of a material composition.

11. Method according to claim 10, wherein the material composition is a plastic film and/or a packaging material

## Revendications

1. Dispositif (18) pour mesurer un taux de transition entre une première phase (1) d'un matériau et une seconde phase (2) du matériau, le matériau étant solide ou liquide dans la première phase (1) et gazeux dans la seconde phase (2), comprenant:
une plaque de substrat poreux (20) pour générer artificiellement ladite première phase (1), ladite plaque de substrat poreux (20) ayant une surface inférieure (21) et une surface supérieure (22); et
une chaîne de thermocouples (4) fixée à la plaque de substrat poreuse (20) et ayant une pluralité de jonctions conductrices (6), la chaîne de thermocouples (4) ayant une première zone (7) comprenant chaque seconde des jonctions conductrices (6) et une seconde zone (8) comprenant les jonctions conductrices restantes (6) et étant adaptée pour délivrer une tension thermoélectrique mesurable représentant une différence de température entre les première et seconde zones (7, 8) de la chaîne de thermocouples (4), dans laquelle
soit la chaîne thermique (4) est située entièrement sur le côté inférieur (21) ou sur le côté supérieur (22) de la plaque de substrat (20) et la première partie (7) de la chaîne thermique (4) est située plus près du côté correspondant (12, 22) de la plaque de substrat (20) que la deuxième partie (8) de la chaîne thermique (4),
soit la première zone (7) et la deuxième zone (8) de la chaîne thermique (4) sont situées dans un plan commun parallèle à la face inférieure (21) et/ou à la face supérieure (22) de la plaque de substrat (20) et il est prévu un couvercle (10) qui recouvre la plaque de substrat poreuse (20) au niveau de la première zone (7) de la chaîne thermique (4) mais pas au niveau de la seconde zone (8) de la chaîne thermique (4).

2. Dispositif selon la revendication 1, qui comprend en outre un récipient (14) avec un fond (30), dans lequel la plaque de substrat poreuse (20) peut être montée dans le récipient (14) de telle sorte qu'elle divise le volume de réception du récipient (14) en une partie inférieure (14a) comprenant le fond (30) et une partie supérieure (14b).

3. Dispositif selon la revendication 1, qui comprend en outre un tube (34), dans lequel la plaque de substrat poreuse (20) est montée dans le tube (34) de telle manière qu'elle divise le tube (34) en deux sections se succédant axialement.

4. Dispositif selon l'une quelconque des revendications précédentes, comprenant en outre un capteur d'humidité et de température (15).

5. Dispositif selon l'une des revendications précédentes, dans lequel la plaque de substrat poreuse (20) présente un diamètre de pore moyen inférieur à 100 µm, inférieur à 50 µm, inférieur à 10 µm, inférieur à 1 µm ou inférieur à 800 nm ou un diamètre de pore moyen compris entre 200 nm et 800 nm, entre 500 nm et 2 µm ou entre 200 nm et 5 µm.

6. Procédé pour mesurer un taux de transition entre une première phase (1) d'un matériau et une seconde phase (2) du matériau, le matériau étant solide ou liquide dans la première phase (1) et gazeux dans la seconde phase (2), comprenant les opérations suivantes:
générer artificiellement la première phase (1) par une plaque de substrat poreuse (20);
fournir une chaîne thermique (4) comprenant une pluralité de jonctions conductrices (6) à une interface (3) entre les première et deuxième phases (1, 2), la chaîne thermique (4) comportant une première zone (7) comprenant chaque deuxième des jonctions conductrices (6) et une seconde zone (8) comprenant les jonctions conductrices restantes (6); et
mesurer une tension thermoélectrique appliquée à la chaîne de thermocouples (4), qui représente une différence de température entre la première et la seconde zone (7, 8) de la chaîne de thermocouples (4).

7. Procédé selon la revendication 6, dans lequel la chaîne thermique (4) est intégrée ou fixée de manière fixe à la plaque de substrat (20).

8. Procédé pour déterminer un potentiel de pression comprenant les opérations suivantes:
mise en oeuvre du procédé selon la revendication 6 ou 7 et
détermination du potentiel de pression de la première phase (1) du matériau ou de la seconde phase du matériau (2) sur la base d'une valeur connue ou mesurée du potentiel de pression de l'autre phase (1, 2) du matériau et de la tension thermoélectrique mesurée.

9. Procédé pour déterminer une perméabilité d'une couche de séparation (12), en particulier d'un film ou d'une couche textile, comprenant:
la réalisation du procédé selon la revendication 6 ou 7, la couche de séparation (12) étant présente entre la première phase (1) et la seconde phase (2);
la déterminatoin de la perméabilité de la couche de séparation (12) sur la base de la contrainte thermique mesurée et des valeurs connues ou mesurées des potentiels de pression des deux phases (1, 2) du matériau.

10. Procédé selon l'une quelconque des revendications 6 à 9, ledit procédé permettant de mesurer un taux de transpiration d'un être vivant, pour mesurer un taux d'évaporation d'un sol (80) ou pour déterminer le potentiel en eau ou la perméabilité à l'eau d'une composition de matière.

11. Procédé selon la revendication 10, dans lequel la composition de la matière est un film et/ou un matériau d'emballage.
